(19) **Europäisches Patentamt / European Patent Office / Office européen des brevets**

(11) **EP 1 946 697 A1**

(12) **EUROPEAN PATENT APPLICATION**

(43) Date of publication:
**23.07.2008 Bulletin 2008/30**

(51) Int Cl.:
***A61B 5/00*** *(2006.01)*

(21) Application number: **07100569.8**

(22) Date of filing: **16.01.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **CSEM Centre Suisse d'Electronique et de Microtechnique SA Recherche et Développement 2007 Neuchâtel (CH)**

(72) Inventors:
• **Sola I Caros, Josep**
  **2035, Corcelles (CH)**
• **Verjus, Christophe**
  **2000, Neuchâtel (CH)**
• **Krauss, Jens**
  **3911, Ried-Brig (CH)**
• **Chetelat, Olivier**
  **2068, Hauterive (CH)**
• **Neuman, Victor**
  **2036, Cormondrèche (CH)**

(74) Representative: **GLN**
  **Rue du Puits-Godet 8a**
  **2000 Neuchâtel (CH)**

(54) **Device for monitoring arterial oxygen saturation**

(57)     The present invention concerns an optical based pulse oximetry device comprising:
- first (10), second (13) and third (16) light emitting means, for placement on the skin surface of a body part to inject light in a tissue of said part, the wavelengths of the light emitted by said second and third means being different from each other
- light detecting means (11, 14, 17) located at a relatively short distance from said first light emitting means (10) and at relatively long distance from said second light emitting means (13) and said third light emitting means (16), for collecting at the skin surface light of said emitting means having travelled through said tissue,
- first computing means (21) for denoising the output signals of said long distance light detecting means (14, 17) from the output signals of said short distance light detecting means (11), and
- second computing means (22) for deriving oximetry measurements from the denoised output signals of said long distance light detecting means (14, 17).

Fig.1

EP 1 946 697 A1

**EP 1 946 697 A1**

## Description

The present invention relates to optical-based pulse oximetry. It concerns, more particularly, a pulse oximetry device for monitoring the oxygen saturation (the so called Sp02) of the haemoglobin in arterial blood.

One very interesting application of the invention is the help of subjects requiring continuous Sp02 monitoring, such as, for example, persons suffering from sleep disturbances, neonates, persons having aerospace and aviation activities, alpinists, high altitude sportmen......

Since the early works of T. Aoyagi, the principles of pulse oximetry have been established (J. G. Webster, Design of Pulse Oximeters, Institute of Physics Publishing, 1997).1]. Two contrasting wavelength lights (e.g. $\lambda_r$ =660$nm$ and $\lambda_{ir}$ = 940$nm$) are injected in a tissue and a reflected or transmitted part of the photons is further recuperated at the skin surface. The changes in light absorption occurred through the pulsated vascular bed are analysed by means of the Beer-Lambert law. According to this law, the intensity $I$ of light recuperated at the skin surface can be characterized by the expression $I = I_0 e^{\alpha_\lambda d}$ , where $I_0$ denotes the baseline intensity of light and $e^{\alpha_\lambda d}$ models the vascular bed absorption which depends on the absorption index $\alpha_\lambda$ at the wavelength $\lambda$ and the vascular bed thickness d .

Due to cardiac activity, the thickness of the vascular bed continuously evolves ( $d = d_0 + \Delta d(t)$ ) and so does $I$ =$I(t)$ . By identifying a characteristic cardiac pattern in both $I_r$ and $I_{ir}$ , an estimation of the ratio $\alpha_r / \alpha_{ir}$ can be obtained.

Hence, the relative content of oxygenated haemoglobin in the arterial tree is derived by means of an empirically calculated calibration look-up table.

Classical pulse oximeters, one example of which is described in the above-mentioned publication, require the cardiac pattern to be continuously identified and tracked. The apparition of the cardiac activity in the optical intensity is detected by a photo-plethysmograph. The amount of absorbed light correlates with the pulsation of arterial blood, and thus, to the cardiac activity.

In the state-of-the-art, two types of Sp02 probes are currently used, namely reflectance and transmission probes. Both methods are based on the placement of two light sources (LED) and a light detector (photodiode) on the skin surface.

In transmission probes, the optical elements are located on opposite sides of a body part. This configuration assures an easy detection of pulsatile patterns but limits considerably the areas of the body where it can be used: finger-tip, ear-lobes and toe.

In reflectance probes, both optical elements are placed at the same plane of a body surface. The recuperated light is, in this case, backscattered in the body and collected at the skin surface. This configuration virtually allows locating the Sp02 probe at any body placement but creates a severe limitation on its ambulatory use. The probe design must eliminate the possibility of direct light passing from the optical source to the photo-detector (cross-talk or optical shunt). Up-to-date, this limitation has been solved either by glue-fixing the probe to the skin or by means of vacuum techniques. An alternative approach is to further separate the optical components. Hence, the probability of cross-talk is considerably reduced. However, due to the enlarged light-path, a drastic decrease of the received light power is obtained and the detection of pulsatile light becomes troublesome. Some manufacturers have proposed the use of the ECG as an additional recording to overcome such limitations.

It is an object of this invention to provide a device for monitoring arterial oxygen saturation that extends the use of reflectance optical-probes to any body location by reducing fixation constrains. Even more, the method overcomes the requirement of an auxiliary ECG recording and restricts the probe to an optical-only-sensor.

More precisely, the present invention concerns an optical based pulse oximetry device comprising:

- first, second and third light emitting means, for placement on the skin surface of a body part to inject light in a tissue of said part, the wavelengths of the light emitted by said second and third means being different from each other,
- light detecting means located at a relatively short distance from said first light emitting means and at relatively long distance from said second light emitting means and said third light emitting means, for collecting at the skin surface light of said emitting means having travelled through said tissue,
- first computing means for denoising the output signals of said long distance light detecting means from the output signals of said short distance light detecting means, and
- second computing means for deriving oximetry measurements from the denoised output signals of said long distance light detecting means.

According to a first preferred embodiment of the invention, said first computing means is programmed to detect the temporal positions of every maximum of the output signals of said short distance light detecting means, then to perform, from the sequence of the detected maximum positions, a triggered averaging of the output signals of said long distance light detecting means.

According to a second preferred embodiment of the invention, said first computing means is programmed to estimate a representation of the spectral distribution of the output signals of said short distance light detecting means, then to perform, from said estimated representation, the restoring of the output signals of said long distance light detecting means.

Other features and objects of the present invention will become more apparent by reference to the following description taken in conjunction with the attached drawings, in which:

- figures 1 and 4 are block diagrams of two preferred embodiments of an optical based pulse oximetry device according to this invention; and
- figures 2 and 3 show two examples of placement of the light emitting means and the light detecting means at the skin surface.

When performing reflectance pulse oximetry, two main reasons justify the increase of the physical separation between optical parts (LEDs and photo-diode).

1. In any pulse oximetry probe, the relative pulse amplitude is a good indicator of the quality of the probe placement. This quality factor, usually depicted as Perfusion Index (PI), is also interpreted as a quantification of the width of the vascular bed traversed by a light beam. It was demonstrated (Y. Mendelson, Noninvasive Pulse Oximetry Utilizing Skin Reflectance Photoplethysmography, IEEE Transactions on Biomedical Engineering, Vol 35, No 10, 1988) that, given a reflectance probe, the PI is linearly increasing with the increase of the physical separation between the optical parts.

2. The probability of direct-light being short-cut from the light sources to the light detector by scattering in the outer part of the skin or/and successive reflection in the probe-skin interface is reduced when both optical elements are dispersed. Due to the reduced light short-cut, probe design can be simplified (no glue fixing is required anymore).

However, by increasing the distance between the optical parts, the absolute intensity of received light at the light detector is exponentially decreased and, thus, the quantification of the pulsatile signal becomes problematic, compromising the feasibility of successfully identifying cardiac activity. In the state-of-the-art reflectance probes, the facts here exposed have imposed a trade-off between:

- Increasing the physical separation of optical elements, thus reducing cross-talk and increasing the Perfusion Index (PI).
- Assuring enough light intensity at the photo-detector.

This trade-off has historically forced transmittance probes to include severe fixing mechanism such as glue or vacuum approaches, as described in the already mentioned J. G. Webster publication or by V. Konig (Reflectance Pulse Oximetry - Principles and Obstetric Application in the Zurich System, Journal of Clinical Monitoring and Computing 14: 403-412, 1998).

The following table summarizes the advantages and disadvantages of near and far-field photo-plethysmography:

| Separation | Received Light Intensity | Perfusion Index (PI) | Cross-talk | Utility |
|---|---|---|---|---|
| Near | Excellent | Poor | Likely | Easy detection of cardiac activity |
| Far | Poor | Excellent | Unlikely | Reliable pulse oximetry estimations |

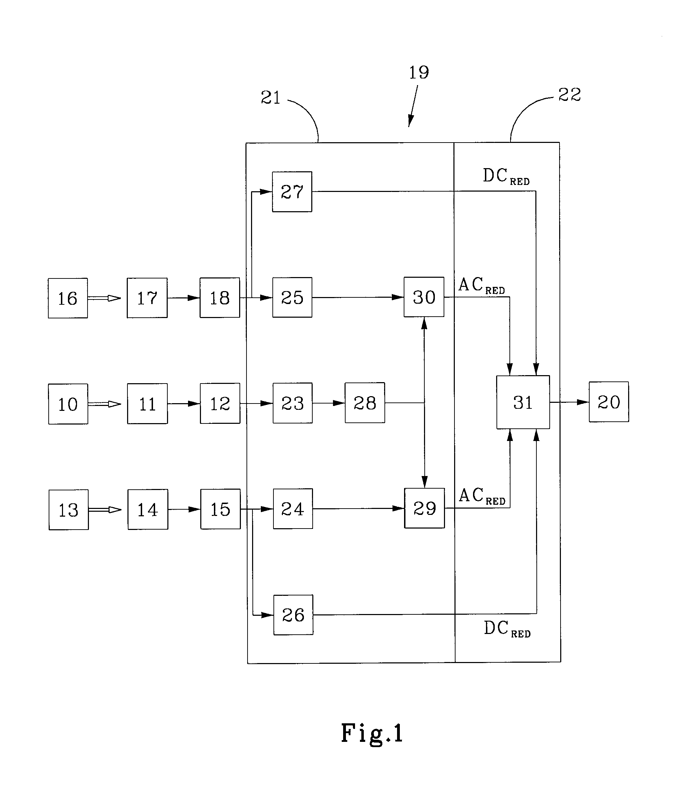

The present invention merges the advantages of both near and far field photo-plethysmography in a single method. As shown in figure 1, the invention consists in combining far and near photo-plethysmographs so that:

- a near-field photo-plethysmograph allows the continuous tracking/detection of cardiac activity;
- a far-field photo-plethysmograph performs pulse oximetry measurements on the basis of estimated cardiac activity information.

According to the invention, a near-field reflectance photo-plethysmograph and a far-field reflectance photo-plethysmograph are merged in a unique device comprising:

- for the near-field function, a first light source 10, which can be a LED emitting in the infra-red range at 940nm, a first light detector 11, such as a photo-diode, located to receive light from the source, and a first analog-to-digital converter (ADC) 12 connected at the output of the light detector;
- for the far-field function, a second light source 13, such as a LED, emitting in the infra-red range at 940nm, a second light detector 14, such as a photo-diode, located to receive light from source 13, a second analog-to-digital converter (ADC) 15 connected at the output light detector 14, a third light source 16, such as a LED, emitting in the red range at 660nm, a third light detector 17, such as a photo-diode, located to receive light from source 16, and a third analog-to-digital converter (ADC) 18 connected at the output of light detector 17;
- a microprocessor 19 connected at the outputs of analog-to-digital converters 12, 15 and 18; and .
- a display device 20 connected at the output of microprocessor 19.

The above-mentioned wavelength values of 660 and 940nm are just given as examples. More generally, these wavelengths must be in the visible infra-red region, i.e comprised between 400 and 2000nm, and be different from each other.

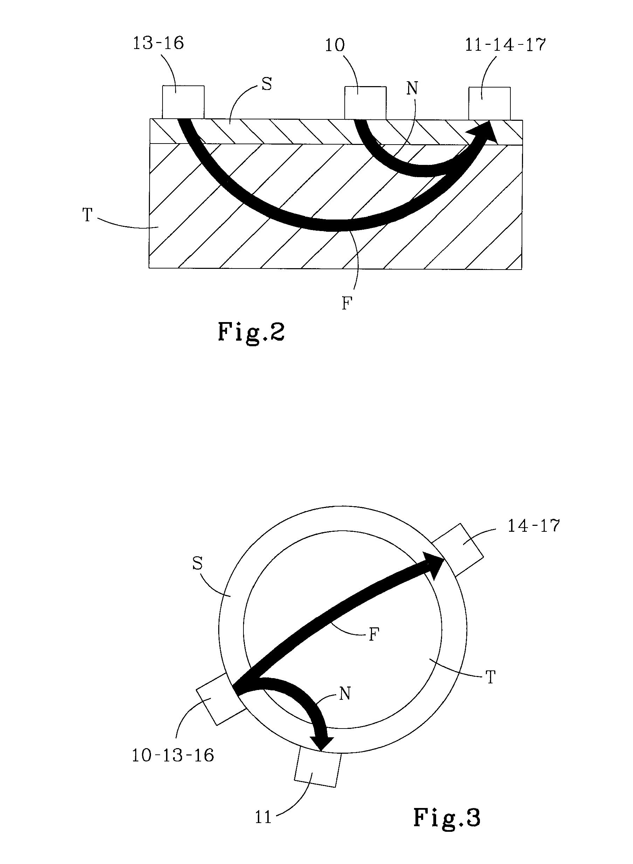

As shown in figure 2, light sources 10-13-16 and light detectors 11-14-17 are positioned at the surface of the skin S of a body part. The light detectors are at the same location. Near-field light source 10 is at a shorter distance from the detectors than far-field light sources 13-16, located at the same place.

Typically, the separation between the near-field light source and the light detectors is between 4 and 10mm, whereas the separation between the far-field light sources and the light detectors is between 10 and 50mm.

Figure 2 shows that the light collected by the detectors has travelled in tissue T longer and deeper for the far-field beam F than for the near-field beam N.

The above described structure is a simplified presentation of the device of the invention. Needless to mention that a single light detector and a single analog-to-digital converter can also be used in association with time-sharing control means adapted to apply to microprocessor 19 data corresponding respectively to the three light sources 10, 13 and 16.

According to the present invention, the light sources and the light detectors can be arranged at the skin surface in many different configurations, the only rule to respect being:

- to collect a light beam having travelled over a short distance in the body, and
- to collect two light beams of different wavelengths in the visible infra-red region having travelled over a longer distance in the body.

Thus, for example, the three light sources can be located at the same place, with a near-field detector at short distance and far-field detectors at longer distance.

Another example is to have a plurality of light sources distributed around far-field detectors, with a near-field detector located at a shorter distance from one of the sources.

Figure 3 shows, as a further example (with the same reference letters as figure 2), that the device of the invention can be arranged around the finger of a person. In that case, light sources 10-13-16 are located at the same place, near-field detector 11 is near the sources and far-field detectors 14-17 stand opposite to the light sources.

Microprocessor 19 has the following two functions:

- Stage 21

  Due to the increased distance between light sources 13-16 and far light detectors 14-17, the digital signals provided by far-field analog-to-digital converters 15 and 18 are noise polluted and render very difficult a reliable identification of the cardiac activity. But the reduced distance separating light source 10 and near light detector 11 assures enough received light intensity and provides a much better identification of the cardiac activity. In stage 21, the near-field signals are used, therefore, to base the pulse oximetry measurements on an improved far-field information.

- Stage 22

  The signals provided by stage 21 are finally used for conventional pulse oximetry calculations.

As shown in figure 1, the digital output of near-field ADC 12 is first applied to a band-pass filter 23, such as a Chebyshev filter Type 1, $3^{rd}$ order, having a band-pass of 0,5 to 3,5 Hz. Knowing that the useful portion of the signal corresponds to the normal, around 1 Hz, cardiac frequency of a person, this filter eliminates the portions of the signal which are outside the 0,5-3,5 Hz range.

Similarly, the digital outputs of infra-red far-field ADC 15 and of red far-field ADC 18 are first applied respectively to band-pass filters 24 and 25, identical to band-pass filter 23.

In addition, the digital outputs of infra-red far-field ADC 15 and of red far-field ADC 18 are applied respectively to identical low-pass filters 26 and 27, such as Butterworth filters, $2^{nd}$ order, which have the function to eliminate the portion of the received signals above 0,2 Hz. The remaining portion of the signals are taken respectively as the DC-infra-red ($DC_{ired}$) and the DC-red ($DC_{red}$) components of the far-field signals.

The operation shown in 28 is the detection of the temporal position of every maximum of the signal delivered by band-pass filter 23. The sequence of the maximum position is then used to perform, respectively in 29 and 30, a triggered averaging of the infra-red and red far-field signals produced by band-pass filters 24 and 25. The triggered averaging is performed in a similar way to that described in the already mentioned publication of J. G. Webster. The triggered averaged signals resulting from operations 29 and 30 are taken respectively as the AC-infra-red ($AC_{ired}$) and the AC-red ($AC_{red}$) components of the far-field signals.

Finally, in stage 22, the $DC_{ired}$, $DC_{red}$, $AC_{ired}$ and $AC_{red}$ signals are used to perform classical pulse oximetry calculations 31, as described by J. G. Webster. The results of the calculations are displayed by device 20 connected at the output of microprocessor 19.

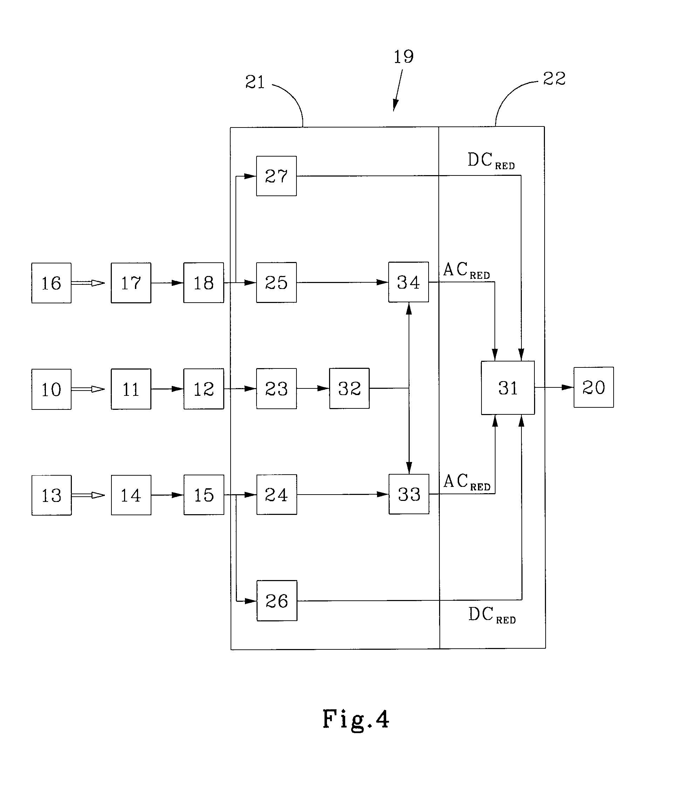

Reference is made, now, to figure 4 which presents another method for obtaining pulse oximetry measurements from the signals delivered by band-pass filters 23, 24 and 25 and by low-pass filters 26 and 27. The elements common to the device of figure 1 are designated by the same references

As shown in figure 4, the near-field signals produced by band-pass filter 23 are used, in 32, to estimate a a-priori representation of the spectral distribution of the cardiac activity, as disclosed, for example, in the publication of D. G. Manolakis, Statistical and Adaptive Signal Processing, McGraw-Hill Higher Education, 2000.

Then, the estimated representation of the spectral distribution of the cardiac activity is used, respectively in 33 and 34, to denoise and/or restore the corrupted infra-red and red far-field signals produced by band-pass filters 24 and 25. The technique used is described, for example, in the already mentioned publication of D. G. Manolakis. The restored signals resulting from operations 33 and 34 are taken respectively as the AC-infra-red ($AC_{ired}$) and the AC-red ($AC_{red}$) components of the far-field signals. They are finally used to perform the classical pulse oximetry calculations 31.

The present invention can be used in many optical-based pulse oximetry applications. For example, a probe carrying the light sources and the light detectors can be placed:

- in a head band, the frontal bone acting as reflectance surface;
- in a mask, the maxillary bone acting as reflectance surface;
- in a chest-belt, the manubrium acting as reflectance surface;
- around a finger;
- around the leg or arm of a neonate;
- as a ear-phone.

**Claims**

1. An optical based pulse oximetry device comprising:

   - first (10), second (13) and third (16) light emitting means, for placement on the skin surface of a body part to inject light in a tissue of said part, the wavelengths of the light emitted by said second and third means being different from each other
   - light detecting means (11, 14, 17) located at a relatively short distance from said first light emitting means (10) and at relatively long distance from said second light emitting means (13) and said third light emitting means (16), for collecting at the skin surface light of said emitting means having travelled through said tissue,
   - first computing means (21) for denoising the output signals of said long distance light detecting means (14, 17) from the output signals of said short distance light detecting means (11), and
   - second computing means (22) for deriving oximetry measurements from the denoised output signals of said long distance light detecting means (14, 17).

2. The device of claim 1, wherein the wavelength of the light of said second (13) and third (16) light emitting means is in the visible infra-red region.

3. The device of claim 1, wherein said short distance is comprised between 4 and 10mm and said long distance is comprised between 10 and 50mm.

4. The device of claim 1, further comprising::

   - bandpass filters (23, 24, 25) connected between said light detecting means and said first computing means, and
   - lowpass fiters (26, 27) connected between said long distance light detecting means and said second computing means.

5. The device of claim 4, wherein said bandpass filters eliminate the portions of the received signals which are outside the 0,5-3,5 Hz range.

6. The device of claim 4, wherein said lowpass filters eliminate the portions of the received signals which are above 0,2 Hz.

7. The device of claim 1, wherein said first computing means (21) is programmed to detect the temporal positions of every maximum of the output signals of said short distance light detecting means, then to perform, from the sequence of the detected maximum positions, a triggered averaging of the output signals of said long distance light detecting means.

8. The device of claim 1, wherein said first computing means (21) is programmed to estimate a representation of the spectral distribution of the output signals of said short distance light detecting means, then to perform, from said estimated representation, the restoring of the output signals of said long distance light detecting means.

Fig.1

Fig.2

Fig.3

Fig.4

**European Patent Office**

**EUROPEAN SEARCH REPORT**

Application Number

EP 07 10 0569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 95/12349 A (AARNOUDSE JAN GERARD [NL]; CT VOOR BIOMEDISCHE TECHNOLOGI [NL]; GRAAFF) 11 May 1995 (1995-05-11) | 1,2,4-6 | INV. A61B5/00 |
| Y | * page 5, lines 21-24 * <br> * page 7, line 9 - line 15 * <br> * page 8, line 15 - line 21 * <br> * page 17, line 15 - page 21, line 31 * <br> * page 21, line 33 - page 26, line 36 * <br> * page 28, line 11 - line 23 * <br> * claims 1-3 * <br> * figures 3,4,7 * | 8 | |
| Y | US 5 490 505 A (DIAB MOHAMED K [US] ET AL) 13 February 1996 (1996-02-13) <br> * abstract * <br> * page 49, line 39 - line 48 * | 8 | |
| X | US 5 299 570 A (HATSCHEK RUDOLF A [CH]) 5 April 1994 (1994-04-05) <br> * column 8, lines 13-38 * <br> * column 9, lines 12-16 * <br> * column 10, line 8 - line 24 * <br> * column 18, line 12 - line 17 * <br> * figure 2 * | 1-6 | |
| X | EP 0 945 100 A (ISS USA INC [US]) 29 September 1999 (1999-09-29) <br> * paragraphs [0056] - [0066], [0070]; figure 4 * | 1,2,4-6 | TECHNICAL FIELDS SEARCHED (IPC) <br><br> A61B |
| X | EP 0 286 142 A2 (SUMITOMO ELECTRIC INDUSTRIES [JP]) 12 October 1988 (1988-10-12) <br> * page 3, line 14 - page 4, line 7 * <br> * page 4, line 25 - line 38 * <br> * page 5, line 32 - line 40 * <br> * figure 2a * | 1,2,4-6 | |

-/--

The present search report has been drawn up for all claims

| Place of search | Date of completion of the search | Examiner |
|---|---|---|
| The Hague | 3 August 2007 | Bengtsson, Johan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 07 10 0569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/47039 A (AIHONEN JUKKA [FI]; KIRVESKARI JUHA [FI]; PENTTILAE HEIKKI [FI]; SIRKI)<br>23 September 1999 (1999-09-23)<br>* page 3, line 13 - line 21 *<br>* page 8, line 18 - page 9, line 9 *<br>* figure 6b *<br>----- | 1,2,4-7 | |
| A | WO 03/103486 A (MASSACHUSETTS INST TECHNOLOGY [US])<br>18 December 2003 (2003-12-18)<br>* the whole document *<br>----- | 1,2,8 | |
| A | ASADA H H ET AL: "Mobile monitoring with wearable photoplethysmographic biosensors"<br>IEEE ENGINEERING IN MEDICINE AND BIOLOGY MAGAZINE, IEEE SERVICE CENTER, PISACATAWAY, NJ, US,<br>vol. 22, no. 3, May 2003 (2003-05), pages 28-40, XP011098587<br>ISSN: 0739-5175<br>* the whole document *<br>----- | 1,2,8 | TECHNICAL FIELDS SEARCHED (IPC) |

The present search report has been drawn up for all claims

| Place of search | Date of completion of the search | Examiner |
|---|---|---|
| The Hague | 3 August 2007 | Bengtsson, Johan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                         

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 10 0569

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2007

| Patent document cited in search report | | Publication date | Patent family member(s) | | Publication date |
|---|---|---|---|---|---|
| WO 9512349 | A | 11-05-1995 | NONE | | |
| US 5490505 | A | 13-02-1996 | US | 5685299 A | 11-11-1997 |
| | | | US | 2004236196 A1 | 25-11-2004 |
| | | | US | 6263222 B1 | 17-07-2001 |
| US 5299570 | A | 05-04-1994 | AT | 124225 T | 15-07-1995 |
| | | | DE | 59202684 D1 | 03-08-1995 |
| | | | EP | 0527703 A1 | 17-02-1993 |
| | | | JP | 2588670 B2 | 05-03-1997 |
| | | | JP | 5192316 A | 03-08-1993 |
| EP 0945100 | A | 29-09-1999 | CA | 2266284 A1 | 25-09-1999 |
| | | | JP | 11344442 A | 14-12-1999 |
| | | | US | 6078833 A | 20-06-2000 |
| EP 0286142 | A2 | 12-10-1988 | DE | 3851251 D1 | 06-10-1994 |
| | | | DE | 3851251 T2 | 15-12-1994 |
| | | | JP | 63252239 A | 19-10-1988 |
| | | | US | 4867557 A | 19-09-1989 |
| WO 9947039 | A | 23-09-1999 | NONE | | |
| WO 03103486 | A | 18-12-2003 | AU | 2003232450 A1 | 22-12-2003 |

EPO FORM P0459

For more details about this annex : see Official Journal of the European Patent Office, No. 12/82

**REFERENCES CITED IN THE DESCRIPTION**

**Non-patent literature cited in the description**

- **J. G. WEBSTER.** Design of Pulse Oximeters. Institute of Physics Publishing, 1997, vol. 1
- **Y. MENDELSON.** Noninvasive Pulse Oximetry Utilizing Skin Reflectance Photoplethysmography. *IEEE Transactions on Biomedical Engineering,* 1988, vol. 35 (10
- **J. G. WEBSTER ; V. KONIG.** Reflectance Pulse Oximetry - Principles and Obstetric Application in the Zurich System. *Journal of Clinical Monitoring and Computing,* 1998, vol. 14, 403-412
- **D. G. MANOLAKIS.** Statistical and Adaptive Signal Processing. McGraw-Hill Higher Education, 2000